# EUROPEAN PATENT APPLICATION

(11) **EP 2 327 671 A1**
(43) Date of publication of application: **01.06.2011**
(21) Application number: 09793975.5
(22) Date of filing: 09.07.2009
(51) Int. Cl.: C04B 22/06, C04B 22/12, C04B 22/16

(54) **CEMENT-SETTING-TIME ACCELERATOR COMPOSITIONS**

(30) Priority: 10.07.2008 ES 200802095; 21.05.2009 ES 200901292
(71) Applicant: Universitat Autonoma de Barcelona, 08193 Bellaterra (ES); The Research Foundation Of State University Of New York, Amherst, NY 14228 (US)
(72) Inventor: MUÑOZ VIVEROS, Carlos A., Amherst, New York 14228 (US); CAMPILLO FUNOLET, Marc, E-08193 Bellaterra (ES); TORRADO BONALS. Anna, E-08193 Bellaterra (ES); VALIENTE MALMAGRO, Manuel, E-08193 Bellaterra (ES)
(74) Representative: Carvajal y Urquijo, Isabel
(86) International application number: PCT/ES2009/000359
(87) International publication number: WO 2010/004067

(57) **Abstract**

The invention refers to setting time accelerator compositions for Portland type cements comprising at least two of the following compounds: a) CaCl2, b) Phosphate, c) Silica, as well as their use in dental coating.

## Description

### Field of the invention

The present invention refers in general to compositions comprising additives for cement compounds and it particularly refers to compositions comprising additives accelerating the setting time of cements.

### State of the art

Mineral Trioxide Aggregate (MTA) is a byproduct of Portland cement comprising mainly tricalcium silicate, tricalcium aluminum, tricalcium oxide and silicate oxide (Torabinejad M, Hong CU, McDonald F, Pitt Ford TR. "Physical and chemical properties of a new root-end filling material". J Endodont 1995; 21:349-53).

Given its high biocompatibility and good sealing capacity, MTA is being widely used in dentistry, as described in US patent 5,769,638. In the last few years, several studies have been published showing that MTA cement promotes the tissue regeneration in contact with dental pulp and of periradicular tissues, so that for many researchers this material has become the ideal dental material. However, MTA has a long setting period, which entails a problem for its dental application as it produces discomfort in patients.

### Description of the invention

The present invention solves the aforementioned problem since it provides a composition which when added to Portland type cement and MTA considerably reduces the setting time.

Thus, in a first aspect, the present invention refers to a setting time accelerator composition for Portland type cement comprising at least two of the following compounds:
- CaCl₂
- Phosphate
- Silica

In the present invention when we refer to silica, we refer to any form of silicon dioxide, we particularly refer to any form of amorphous silicon dioxide and more particularly, we refer to any form of hydrophobic pyrogenic silicon dioxide.

In the present invention, when we refer to phosphate we refer to any phosphoric acid salt.

In another aspect, the invention refers to a composition comprising CaHPO₄ as phosphate.

In a particular aspect, the composition of the present invention comprises CaCl₂ and CaHPO₄;

In another particular aspect, the composition of the present invention comprises CaCl₂ and silica;

In another particular aspect, the composition of the present invention comprises CaHPO₄ and silica.

In a more particular aspect, the composition of the present invention comprises a maximum weight of:
- 20% of CaCl₂ with reference to the total amount of cement, more particularly the maximum amount of CaCl₂ is 12% with reference to the total amount of cement, more particularly the maximum amount of CaCl₂ is 9% with reference to the total amount of cement,
- 20% of CaHPO₄ with reference to the total amount of cement, more particularly the maximum amount of CaHPO₄ is 14% with reference to the total amount of cement, more particularly the maximum amount of CaHPO₄ is 10% with reference to the total amount of cement.

In a more particular aspect, the composition of the present invention comprises a maximum weight of:
- 20% of CaCl₂ with reference to the total amount of cement, more particularly the maximum amount of CaCl₂ is 12% with reference to the total amount of cement, more particularly the maximum amount of CaCl₂ is 9% with reference to the total amount of cement,
- 10% of silica with reference to the total amount of cement, more particularly the maximum amount of silica is 3% with reference to the total amount of cement, more particularly the maximum amount of silica is 2% with reference to the total amount of cement.

In a more particular aspect, the composition of the present invention comprises a maximum weight of:
- 20% of CaHPO₄ with reference to the total amount of cement, more particularly the maximum amount of CaHPO₄ is 14% with reference to the total amount of cement, more particularly the maximum amount of CaHPO₄ is 10% with reference to the total amount of cement,
- 10% of silica with reference to the total amount of cement, more particularly the maximum amount of silica is 3% with reference to the total amount of cement, more particularly the maximum amount of silica is 2% with reference to the total amount of cement.

In a particular aspect, the silica of the composition of the present invention is US202.

In a particular aspect of the present invention, the compounds which form part of the setting time accelerator composition are in the form of powder.

In another particular aspect, the compounds which form part of the setting time accelerator composition are in the form of an aqueous solution.

In another particular aspect of the invention, the compounds which form part of the setting time accelerator composition are in the form of an aqueous suspension.

When we refer to Portland type cement in the present invention, we refer to a cement of hydraulic material based on tricalcium silicate and dicalcium silicate, besides other components, such as barium salts, calcium aluminate, magnesium dioxide, etc.

In a particular aspect of the present invention, the Portland type cement is MTA cement.

In the present invention setting time acceleration refers to a reduction of the setting time for each cement with respect to the original setting time. Particularly, we refer to an initial setting time which is less than 34 minutes and a final setting time which is less than 200 minutes.

In another aspect, the present invention refers to the use of a composition as described before in dental coatings.

### Detailed description of the invention

### Example 1

In order to carry out the present invention Portland cement (Conesland bricolaje, Agroquimica del Vallès S.A., Spain) was used, which has the same setting time as MTA, instead of MTA to reduce testing procedural costs. The composition is the same in both cements except for the fact that the MTA is subjected to a sterilization process.

The additives tested were: CaCl₂·2H₂O (Panreac S.A., Spain), CAHPO₄-2H₂O (Panreac S.A., Spain), and two silicates: TS530 (pyrogenic silicon dioxide) (Degusta Iberia S.A.) and US202 (hydrophobic silicon dioxide) (Degusta Iberia S.A.).

A full factorial 2⁴ design with two experimental levels was used. The samples were prepared weighing the solid additives and the Portland cement and mixing them in a rotating mixer for 60 minutes, in order to attain a homogenous distribution of the mixture components. The data treatment and system modelling were performed using Modde 5.0 software (Umetrics).

The samples were mixed with water using a 3:1 powder:liquid ratio and both the initial setting time and the final setting time were evaluated using Gillmore needles.

The value which was used in the experimental design study was the average of three reproduced measurements.

Table 1 shows the compositions of the samples studied (weight % of each additive with respect to the cement) and the values determined for initial and final setting times.

| **Composition % of the sample with respect to cement** | | | | | **Setting time** | |
|---|---|---|---|---|---|---|
| **Cement (g)** | **%CaCl2** | **%US202** | **%TS530** | **%CaHP04** | **initial (min)** | **final (min)** |
| 3 | 0.00% | 0 | 0 | 0 | 37 | 166 |
| 3.3687 | 9.03% | 0.00% | 0.00% | 0.00% | 19 | 82 |
| 3.0663 | 0.00% | 2.94% | 0.00% | 0.00% | 39 | 195 |
| 3.0032 | 10.16% | 2.54% | 0.00% | 0.00% | 9 | 50 |
| 3.0147 | 0.00% | 0.00% | 2.54% | 0.00% | 29 | 176 |
| 3.1792 | 9.74% | 0.00% | 2.66% | 0.00% | 12 | 69 |
| 3.1066 | 0.00% | 2.61% | 2.53% | 0.00% | 42 | 114 |
| 3.0315 | 10.05% | 2.70% | 2.50% | 0.00% | 105 | 310 |
| 3.0682 | 0.00% | 0.00% | 0.00% | 9.80% | 35 | 119 |
| 3.0795 | 9.85% | 0.00% | 0.00% | 12.81 % | 13 | 57 |
| 3.1203 | 0.00% | 2.45% | 0.00% | 11.61 % | 40 | 97 |
| 3.1813 | 9.63% | 2.47% | 0.00% | 10.08% | 4 | 10 |
| 3.1232 | 0.00% | 0.00% | 2.48% | 10.03% | 32 | 113 |
| 3.0126 | 10.55% | 0.00% | 2.61% | 10.18% | 16 | 77 |
| 3.2117 | 0.00% | 2.66% | 2.54% | 9.76% | 26 | 98 |
| 3.1371 | 10.38% | 2.70% | 2.66% | 9.91% | 14 | 64 |
| 3.1936 | 4.99% | 1.23% | 1.24% | 5.48% | 51 | 90 |
| 3.1113 | 4.90% | 1.18% | 1.52% | 7.77% | 52 | 98 |
| 3.18 | 4.92% | 2.66% | 1.28% | 5.09% | 52 | 87 |

The values of the setting time dropped in some of the compositions studied. The initial setting time included values ranging from 4 to 105 minutes and the final setting time ranged from 10 to 30 minutes.

The setting time for the sample with CaCl₂ and both silicate additives was the highest; this can be so because this sample required some more water than the normal powder:water ratio. The data of this sample were excluded.

The data showed the accelerating effect of CaCl₂ on the setting time, having an important influence on the initial and final setting time.

CaHPO₄, as CaCl₂, has an accelerating effect on the setting time, but it mainly affects the final setting time, the two corresponding anions influence the different reactions of the setting process.

The silicates subjected to the test, by themselves do not have direct effects on the setting time, but when they are in the presence of CaCl₂ and/or CaHPO₄, there occurs a synergism which makes the process faster than when they act alone, both CaCl₂ and CaHPO₄.

### Example 2

This study intends to evaluate and optimise the setting time and physical properties of dental cement based on Portland cement (MTA) and of a traditional Portland type cement.

The following cements were used for the study:
1) Pro Root MTA (Dentsply- Tulsa Dental, Tulsa (OK), lot: 01081581, 05003088)
2) Conesland grey cement (Agroquimica del Valles, St Quirze del Valles, Spain)

### Setting time

For each cement, 7 samples of 1g were prepared and mixed with the corresponding compounds to obtain the different compositions of the study, which are detailed in Table 1. The samples prepared were mixed with water in a 3:1 powder: liquid ratio. One or two Teflon rings approximately 4.8mm high, with an external diameter of 11.1 and an internal diameter of 9.5mm were filled with the cement mixes and slightly pressed with glass sample holders. The setting time was measured using the Gillmore needle method.

**Table 1 Accelerator Compositions. Percentages with respect to the cement weight.**

| Composition | CaCl₂ | Phosphate | Silicate |
|---|---|---|---|
| A | 0% | 0% | 0% |
| B | 10% | CaHPO₄-10% | US202-2.5% |
| C | 20% | CaHPO₂-20% | US202-10% |
| D | 20 % | CaHPO4-20 % | 0% |
| E | 20 % | 0% | US202-10% |
| F | 0% | CaHPO₄-20% | US202-10% |
| H | 10 % | Na₂HPO4-10% | US202-2.5% |
| J | 10% | CaHPO₄-10% | EG50-2.5% |
| K | 10 % | Hydroxyapatite- 10% | US202-2.5 % |
| L | 10 % | Hydroxyapatite - 10 % | EG50 - 2.5 % |

### Setting pH

For each cement, 9 samples of 0.3g were mixed with the corresponding compounds to obtain three replicates of compositions A, B and K. The powder samples were mixed with 3ml of water to prevent them from hardening during the measuring and the suspensions obtained were kept in agitation. The pH of each suspension was measured after 5 minutes of agitation.

### Compressive Strength

For each cement, six samples of 1g were prepared and mixed with the corresponding compounds to obtain each one of the compositions shown in Table 1. The samples were mixed with water with a 3:1 powder: liquid ratio. Five Teflon cylinder moulds with a height of 6mm and an internal diameter of 3mm were filled with each mixture. Between 3 and 5 minutes after starting to mix the powder and the water, the molds were submerged in water at 37C. An hour after the mixing with water, the surfaces of the specimens were ground with 240 grit SIC paper. Next, the specimens were extracted from the molds and kept in water at 37C until it was time to test them.

Twenty-four hours after the mixing of cement with water, the specimens were tested in compressive strength at 1mm/min. The strength values were registered and the compressive strength in MPa was calculated from the values obtained.

The formulation C, D, E and F could not be mixed with water in the 3:1 powder: liquid ratio established. After agitating the samples for 5 minutes, the powder was not wet yet and it was not incorporated in the water. These samples were discarded for the two cements studied.

It was also observed that the samples containing the US202 silicate were much more difficult to mix than the original cement, while the samples prepared with EG50 could be easily mixed and a homogenous mixture was obtained fast.

### Setting time

As shown in Table 2, most formulations tested improved the final setting time of MTA and all except the formulation H improved the setting time of traditional Portland cement.

**Table 2 Setting time mean values**

| **Portland Cement:** Setting time(min) | | | | | | | | | | | |
|---|---|---|---|---|---|---|---|---|---|---|---|
| A | | B | | H | | J | | K | | L | |
| Initial | Final | Initial | Final | Initial | Final | Initial | Final | Initial | Final | Initial | Final |
| 17 | 100 | 27 | 83 | 12 | 215 | 28.5 | 53 | 28 | 60.5 | 26 | 38 |

| **MTA:** Setting time (min) | | | | | | | | | | | |
|---|---|---|---|---|---|---|---|---|---|---|---|
| A | | B | | H | | J | | K | | L | |
| Initial | Final | Initial | Final | Initial | Final | Initial | Final | Initial | Final | Initial | Final |
| 30 | 81 | 12 | 51 | 33 | 67 | 12 | 29 | 11 | 33 | 7 | 18 |

### Setting pH

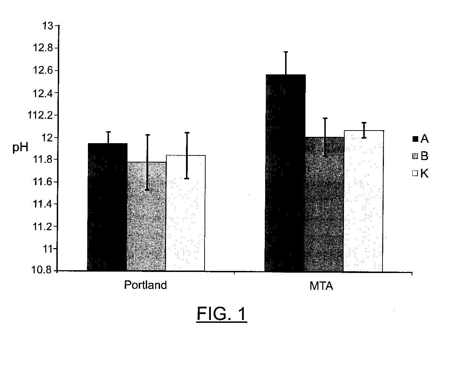

The additives tested slightly reduced the setting pH of MTA, while they did not alter the pH of traditional Portland cement. Figure 1 shows the pH values obtained for each one of the compositions tested.

### Compressive Strength

A minimum of one or two specimens of each group were tested with small defects and could not be tested due to the partial disintegration in water or to the presence of great defects in the walls.

The compressive strength mean values are summarised in Table 3. The composition H produced certain weakening in the two cements while the rest of the compositions increased their compressive strength.

**Table 3 Compression resistance mean values**

| Portland cement compressive strength (MPa) | | | | | |
|---|---|---|---|---|---|
| A | B | H | J | K | L |
| 4.5 | 5.1 | 2.8 | 11.3 | 5.6 | 14.0 |

| MTA compressive strength (MPa) | | | | | |
|---|---|---|---|---|---|
| A | B | H | J | K | L |
| 1.4 | 5.0 | 0.2 | 6.9 | 7.8 | 15.6 |

### Description of the drawings

Figure 1 pH means values after 5 minutes of setting according to the compositions of Example 2.

## Claims

1. A setting time accelerator composition for Portland type cements comprising at least two of the following compounds:
- CaCl2
- Phosphate
- Silica

2. Composition according to claim 1, in which the phosphate is CaHPO₄

3. Composition according to claim 1, wherein the composition comprises a maximum in weight of:
- 20% of CaCl₂ with reference to the total amount of cement,
- 20% of CaHPO₄ wit reference to the total amount of cement,
- 10% in weight of silica with reference to the total amount of cement.

4. Composition according to any of the preceding claims, wherein the composition comprises a maximum in weight of:
- 12% of CaCl₂ with reference to the total amount of cement,
- 14% of CaHPO₄ with reference to the total amount of cement,
- 3% of silica with reference to the total amount of cement.

5. Composition according to any of the preceding claims, wherein the composition comprises:
- 9% of CaCl₂ with reference to the total amount of cement,
- 10% of CaHPO₄ with reference to the total amount of cement,
- 2% in weight of silica with reference to the total amount of cement.

6. Composition according to any of the preceding claims, wherein the silica is US202.

7. Composition according to any of the preceding claims, wherein the composition is in the form of powder.

8. Composition according to any of the claims 1-5, wherein the composition is in the form of an aqueous solution.

9. Composition according to any of the claims 1-5, wherein the composition is in the form of an aqueous suspension.

10. Composition according to any of the preceding claims, wherein the Portland type cement is MTA.

11. Use of a composition according to any of the preceding claims in dental coating.
